(19) Europäisches Patentamt
European Patent Office
Office européen des brevets

(11) Numéro de publication : **0 398 789 B1**

# (12) FASCICULE DE BREVET EUROPEEN

(45) Date de publication du fascicule du brevet : **09.12.92 Bulletin 92/50**

(51) Int. Cl.$^5$ : **A61K 31/135**, A61K 9/12, A61K 9/72

(21) Numéro de dépôt : **90401247.3**

(22) Date de dépôt : **10.05.90**

## (54) Composition administrable par inhalation à base de méthoxamine.

(30) Priorité : **16.05.89 FR 8906380**

(43) Date de publication de la demande : **22.11.90 Bulletin 90/47**

(45) Mention de la délivrance du brevet : **09.12.92 Bulletin 92/50**

(84) Etats contractants désignés : **AT BE CH DE DK FR GB IT LI LU NL SE**

(56) Documents cités :
**US-A- 3 891 757**
**DIALOG INFORMATION SERVICES, Banque de Données: MEDLINE, accès no. 89238446; L.R. CABANES et al.: "Bronchialhyperresponsiveness to methacholine in patients with impaired left ventricular function", & N. ENGL. J. MED. (UNITED STATES)18-05-1989, 320 (20) p. 1317-22**

(73) Titulaire : **UNIVERSITE DE PARIS V**
**12 rue de l'Ecole de Médecine**
**F-75006 Paris (FR)**

(72) Inventeur : **Cabanes, Laure**
**10 rue Dulac**
**F-75015 Paris (FR)**
Inventeur : **Lockhart, Alain**
**150 Avenue du Maine**
**F-75014 Paris (FR)**
Inventeur : **Weber, Simon**
**32 rue de Lagny**
**F-75020 Paris (FR)**

(74) Mandataire : **Phélip, Bruno et al**
**c/o Cabinet Harlé & Phélip 21, rue de La Rochefoucauld**
**F-75009 Paris (FR)**

EP 0 398 789 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une nouvelle application thérapeutique d'un composé appartenant à la classe des alpha-stimulants vasoconstricteurs, et de ses sels pharmaceutiquement acceptables, ce composé étant l'amino-2 (diméthoxy-2,5 phényl)-1 propanol-1, représenté par la formule :

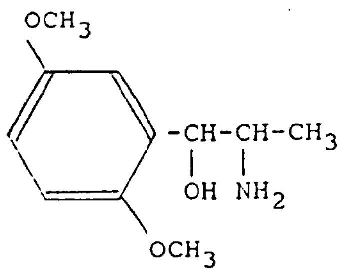

,

et connu sous la Dénomination Commune Internationale de méthoxamine. Pour cette nouvelle indication thérapeutique selon l'invention, qui est celle du traitement et de la prévention des symptômes de l'insuffisance cardiaque, la méthoxamine et ses sels sont présentés sous une nouvelle forme galénique et appliqués suivant une nouvelle voie d'administration.

En partant de l'observation de symptômes d'obstruction bronchique chez les malades présentant une insuffisance cardiaque, les présents inventeurs ont d'abord montré que l'administration, à des patients présentant une insuffisance ventriculaire gauche, de l'agoniste cholinergique métacholine, provoquait une obstruction bronchique à des doses identiques a celles actives chez l'asthmatique, cet effet n'étant pas observé chez des patients présentant une insuffisance coronaire, mais une fonction ventriculaire gauche normale. Sachant que les vaisseaux qui irriguent les bronches se drainent dans le coeur gauche, les présents inventeurs ont administré, en prétraitement, par les voies respiratoires (inhalation), de la méthoxamine-HCl, un vascoconstricteur puissant, et ils ont démontré que cette administration de méthoxamine permet de prévenir la brochoconstriction induite par l'inhalation de métacholine chez ces malades porteurs d'une insuffisance ventriculaire gauche. Les inventeurs ont alors démontré que l'administration de méthoxamine par inhalation était susceptible:

- de diminuer la résistance des voies aériennes chez les sujets en insuffisance ventriculaire gauche décompensée (oedème et suboedème pulmonaires); et
- d'augmenter la tolérance à l'effort des malades porteurs d'une insuffisance ventriculaire gauche compensée, que celle-ci soit ou non d'origine ischémique.

La présente invention a donc pour objet l'utilisation à titre de principe actif de l'amino-2 (diméthoxy-2,5 phényl)-1 propanol-1 et de ses sels pharmaceutiquement acceptables pour fabriquer une composition médicamenteuse se présentant sous une forme administrable par inhalation, pour le traitement et la prévention des symptômes de l'insuffisance cardiaque.

Sont donc concernés par l'application thérapeutique de la présente invention, la méthoxamine et ses sels pharmaceutiquement acceptables avec des acides et dérivés d'ammonium quaternaire. Comme sels d'addition avec les acides, on peut mentionner les sels qui sont formés avec des acides minéraux, tels que l'acide chlorhydrique, l'acide bromhydrique, l'acide sulfurique, l'acide nitrique ou l'acide phosphorique; ainsi que les sels formés avec des acides organiques, tels que l'acide acétique, l'acide propionique, l'acide glycolique, l'acide lactique, l'acide pyruvique, l'acide oxalique, l'acide malonique, l'acide succinique, l'acide malique, l'acide maléique, l'acide fumarique, l'acide tartrique, l'acide citrique, l'acide benzoïque, l'acide cinnamique, l'acide mandélique, l'acide méthane sulfonique, l'acide éthane sulfonique, l'acide p-toluène sulfonique, l'acide salicylique et les acides hydrobenzène sulfoniques. On peut citer plus particulièrement le chlorhydrate de méthoxamine.

La composition médicamenteuse selon l'invention se présente notamment sous la forme d'un aérosol ou d'un nébulisat, la partie non-propulsive de la composition étant constituée par le principe actif en solution physiologique tamponnée à pH neutre ou proche de la neutralité. La concentration du principe actif dans cette solution est comprise entre 1‰ et 10 % (p/v). En particulier, on peut utiliser une concentration se situant dans la plage de 2-5% (p/v).

L'agent propulseur est un gaz pharmaceutiquement acceptable choisi notamment parmi des gaz inertes, comme l'azote ; l'air ; l'oxygène ; les hydrocarbures aliphatiques halogénés, comme le dichlorodifluorométhane ; et leurs mélanges.

Les proportions relatives de la partie non-propulsive au propulseur sont les proportions couramment utilisées par les galéniste pour la préparation des nébulisats et aérosols destinés à l'administration des principes médicamenteux.

La composition selon l'invention est destiné au traitement et à la prévention, chez l'homme et chez l'animal, des symptômes de l'insuffisance ventriculaire gauche, quelle qu'en soit l'étiologie, aussi bien en phase compensée qu'en phase de décompensation. Les diverses indications que l'on peut mentionner sont le traitement et la prévention de la dyspnée d'effort, de la dyspnée de repos, de la dyspnée paroxystique nocturne, et des oedème et sub-oedème pulmonaires.

Le mécanisme de cet effet thérapeutique a pu également être établi :

Lors de l'insuffisance ventriculaire gauche, compensée ou décompensée, se produit, du fait de l'élévation de la pression hydrostatique dans les cavités gauches, une élévation de la pression dans la circulation bronchique responsable d'un épaississement et d'un oedème de la sous-muqueuse bronchique, à son tour responsable d'une obstruction bronchique contribuant à la dyspnée des insuffisants cardiaques. La méthoxamine, alpha-stimulant, du fait de son puissant effet bronchoconstricteur, prévient et corrige cette vasodilatation de la circulation bronchique à l'origine de l'obstruction bronchique. Pour cet effet puisse être bénéfique au plan thérapeutique, il est indispensable d'administrer l'agent alpha-stimulant vasoconstricteur par voie topique (en l'occurrence, par inhalation par le nez ou la bouche), afin de n'obtenir que l'effet local de vasoconstriction de la circulation bronchique sans effet systématique (vasoconstriction généralisée), dont les conséquences hémodynamiques seraient néfastes chez l'insuffisant cardiaque.

La composition selon l'invention pourra être prescrit en traitement continu ou bien alors de façon occasionnelle, par exemple, avant un effort intense de l'insuffisance cardiaque, ce que permet la présentation en récipients pressurisés de type "récipient aérosol", ou nébuliseurs. Concernant la posologie de la composition selon l'invention, on indiquera que les doses à administrer sont variables selon la durée du traitement, la fréquence de la prise, l'hôte, la nature et la gravité de la maladie. On peut mentionner une dose unitaire allant de 0,1 à 100 mg, par exemple une dose unitaire d'environ 10 mg. La posologie quotidienne peut être, à titre d'exemple, de 1 à 10 inhalations par jour, soit 1 à 1000 mg par jour.

Enfin, ladite composition médicamenteuse destinée au traitement et à la prévention des symptômes de l'insuffisance cardiaque peut être obtenue par un procédé caractérisé par le fait qu'on dissout dans une solution physiologique au moins un composé choisi parmi l'amino-2 (diméthoxy-2,5 phényl)-1 propanol-1 et ses sels pharmaceutiquement acceptables, et qu'on place la solution ainsi obtenue sous une forme administrable par inhalation.

Mise en évidence de l'action de la méthoxamine-HCl administrée par inhalation sur la dyspnée des insuffisants cardiaques.

1 - Sélection des patients

Dix patients adultes présentant une grave insuffisance ventriculaire gauche secondaire, due soit à une insuffisance coronaire, soit à une cardiomyopathie dilatée, ont été soumis à cette étude. Tous les patient se trouvaient en classe fonctionnelle NYHA III (New York Heart Association) pour la dyspnée au moment de l'étude, laquelle a été conduite entre septembre 1988 et mars 1989. Tous les patients présentaient une fraction d'éjection ventriculaire gauche, mesurée par angiographie biplane isotopique (à l'aide du technetium $99^{m}$), de moins de 35 pour cent.

Par ailleurs, ces patients présentaient tous un seuil de tolérance à l'effort reproductible défini comme suit :

Les patients ont été soumis deux fois à 24 heures d'intervalle à une même épreuve d'effort de marche sur un tapis roulant, limite par la dyspnée ; il s'agit ici d'un effort à charge constante, à vitesse constante de déroulement du tapis roulant, et à pente constante, connu comme exercice de type rectangulaire. On mesure la durée de marche jusqu'à l'essoufflement du patient. On a choisi des patients dont le seuil de tolérance à l'effort a varié de moins de 30 secondes sur ces deux épreuves.

2 - Déroulement du protocole

Jours 1 et 2 :

Les patients ont été soumis à nouveau aux deux épreuves d'effort afin de vérifier la reproductibilité de la tolérance à l'effort. Les durées d'effort ainsi obtenues représentaient les valeurs de référence.

Jours 3 et 4 :

Les patients ont été chacun soumis aux deux mêmes épreuves d'effort à 24 heures d'intervalle, 15 minutes après avoir reçu, soit du placebo (sérum physiologique seul), soit 10 mg de méthoxamine-HCl (dans la préparation décrite ci-après), par un mode d'administration également décrit ci-après. Cette partie de l'étude a été conduite selon le modèle en cross-over, randomisé et en double aveugle.

3 - Préparation de méthoxamine-HCl

On a préparé une solution à 2% de méthoxamine-HCl dans du sérum physiologique tamponné à pH 7 (solution à 20 mg par millilitre). On a conditionné cette solution dans un nébuliseur Devilbiss 646 (Somerset, Pa.), le propulseur utilisé étant l'oxygène pur.

4 - Mode d'Administration

Les administrations ont été effectuées par le même médecin entre 15h et 18 h au même moment du jour pour un patient donné. La pression sanguine et le débit cardiaque ont été suivis tout au cours des essais à l'aide d'une méthode oscillométrique automatisée (Dinamap TN Critikon).

La préparation de méthoxamine-HCl et le sérum physiologique ont été administrés avec le nébuliseur précité, qui a été activé pendant la phase inspiratoire de la respiration au moyen d'un dosimètre de French-Rosenthal (Laboratory for Applied Immunology, Baltimore) sous une pression d'oxygène pur de 138 kPa. Le temps de nébulisation a été ajusté à environ une seconde, et le nombre de respirations choisi était celui nécessaire pour obtenir la nébulisation de la quantité de 10 mg de méthoxamine HCl (soit 1/2 ml de solution).

5- Résultats

La moyenne des durées d'exercice des deux premières épreuves ($J_1$-$J_2$) a été de 293 secondes ; la durée moyenne d'exercice après placebo a été de 292 secondes, et, après méthoxamine-HCl, de 612 secondes. La méthoxamine-HCl inhalée entraîne donc une augmentation significative, au moins au centième ($p<0,01$), voire au millième, par rapport au placebo, de la durée de l'effort. Ces résultats sont reportés sur le diagramme constituant la figure unique du dessin annexé.

Parallèlement, aucun effet indésirable n'a été observé, et une bonne tolérance du médicament a été notée, aussi bien au plan de la muqueuse bronchique, qu'au plan général.

EXEMPLE DE FORMULATION : Générateur d'aérosol de 20 ml

(A)

| | |
|---|---|
| Méthoxamine-HCl .................... | 100 mg |
| Sérum physiologique tamponné à pH 7 ......... qsp ............... | 5 ml |

(B)

| | |
|---|---|
| Azote .............................. | 15 ml |

## Revendications

1. Utilisation à titre de principe actif de l'amino-2 (diméthoxy-2,5 phényl)-1 propanol-1 et de ses sels pharmaceutiquement acceptables pour fabriquer une composition médicamenteuse se présentant sous une forme administrable par inhalation, pour le traitement et la prévention des symptômes de l'insuffisance cardiaque.

2. Utilisation selon la revendication 1, caractérisée par le fait que le principe actif est le chlorhydrate d'amino-2 (diméthoxy-2,5 phényl)-1 propanol-1 (méthoxamine-HCl).

3. Utilisation selon l'une des revendications 1 et 2, caractérisée par le fait que la composition se présente sous la forme d'un aérosol ou d'un nébulisat, la partie non-propulsive de la composition étant constituée par le principe actif dissous dans une solution physiologique tamponnée à pH neutre ou proche de la neutralité.

4. Utilisation selon la revendication 3, caractérisée par le fait que la concentration du principe actif dans la solution physiologique est comprise entre 1‰ et 10% (p/v).

5. Utilisation selon la revendication 4, caractérisée par le fait que la concentration du principe actif dans la solution physiologique est comprise entre 2% et 5% (p/v).

6. Utilisation selon l'une des revendications 1 à 5, caractérisée par le fait que la composition est administrée à raison de 1 à 1000 mg par jour.

7. Utilisation selon l'une des revendications 1 à 6, caractérisée par le fait qu'on dissout dans une solution physiologique au moins un composé choisi parmi l'amino-2 (diméthoxy-2,5 phényl)- propanol-1 et ses sels pharmaceutiquement acceptables, et qu'on place la solution ainsi obtenue sous une forme administrable par inhalation.

8. Utilisation selon la revendication 7, caractérisée par le fait qu'on prépare une solution physiologique ayant une concentration de 1‰ à 10% (p/v) en principe actif.

9. Utilisation selon la revendication 8, caractérisée par le fait qu'on prépare une solution physiologique ayant une concentration de 2 à 5% (p/v) en principe actif.

## Patentansprüche

1. Verwendung von 2-Amino-1-(2,5-dimethoxyphenyl)-1-propanol und seiner pharmazeutisch annehmbaren Salze als Wirkstoff zur Herstellung einer Arzneizubereitung für inhalatorische Anwendung bei der Behandlung und Vorbeugung von Symptomen der Herzinsuffizienz.

2. Verwendung nach Anspruch 1, dadurch gekennzeichnet, daß der Wirkstoff das Hydrochlorid von 2-Amino-1-(2,5-dimethoxyphenyl)-1-propanol (Methoxamin-HCl) ist.

3. Verwendung gemäß einem der Ansprüche 1 und 2, dadurch gekennzeichnet, daß die Zubereitung als Aerosol oder Vernebelung vorliegt, wobei der nicht-propulsive Anteil der Zubereitung den in einer Physiologischen, auf neutralen, oder nahezu neutralen pH-Wert gepufferten Lösung aufgelösten Wirkstoff umfaßt.

4. Verwendung nach Anspruch 3, dadurch gekennzeichnet, daß die Konzentration des Wirkstoffs in der physiologischen Lösung zwischen 0,1 und 10 Gew./Vol.-% beträgt.

5. Verwendung nach Anspruch 4, dadurch gekennzeichnet, daß die konzentration des Wirkstoffs in der physiologischen Lösung zwischen 2 und 5 Gew./Vol.-% beträgt.

6. Verwendung gemäß einem der Ansprüche 1 bis 5, dadurch gekennzeichnet, daß die Zubereitung in Portionen von 1 bis 1000 mg täglich angewandt wird.

7. Verwendung gemäß einem der Ansprüche 1 bis 6, dadurch gekennzeichnet, daß in einer physiologischen Lösung wenigstens eine aus 2-Amino-1(2,5-dimethoxyphenyl)-1-propanol und seinen pharmazeutish annehmbaren Salzen ausgewählte Verbindung gelöst wird, und daß die so erhaltene Lösung zur inhalatorischen Anwendung bereitgestellt wird.

8. Verwendung gemäß Anspruch 7, dadurch gekennzeichnet, daß eine physiologische Lösung hergestellt wird, die eine konzentration von 0,1 bis 10 Gew./Vol.-% des Wirkstoffs besitzt.

9. Verwendung gemäß Anspruch 8, dadurch gekennzeichnet, daß eine physiologische Lösung hergestellt wird, die eine konzentration von 2 bis 5 Gew./Vol.-% des Wirkstoffs besitzt.

## Claims

1. The use of 2-amino-1-(2,5-dimethoxyphenyl)-1-propanol and its pharmaceutically acceptable salts for

manufacturing a medicinal composition presented in a form which can be administered by inhalation, for the treatment and prevention of the symptoms of heart failure .

2. Use as claimed in claim 1 , characterized in that the active principle is 2-amino-1-(2,5-dimethoxyphenyl)-1-propanol hydrochloride(methoxamine-HCl).

3. Use as claimed in one of claims 1 and 2, characterized in that the composition is presented in the form of an aerosol or a nebulisate, the non-propellent portion of the composition consisting of the active principle dissolved in a physiological solution buffered to neutral pH or a pH close to neutrality .

4. Use as claimed in claim 3, characterized in that the concentration of active principle in the physiological solution is between 0.1 % and 10% (w/v).

5. Use as claimed in claim 4, characterized in that the concentration of active principle in the physiological solution is between 2% and 5%( w/v).

6. Use as claimed in one of claims 1 to 5, characterized in that the composition is administered on the basis of 1 to 1000 mg per day.

7. Use according to one of the claims 1 to 6, characterized in that at least one compound chosen from 2-amino-1-(2,5-dimethoxyphenyl)-1-propanol and its pharmaceutically acceptable salts is dissolved in a physiological solution, and in that the solution thereby obtained is put into a form which can be administered by inhalation.

8. Use as claimed in claim 7, characterized in that a physiological solution having a concentration of 0.1% to 10% (w/v) of active principle is prepared.

9. Use as claimed in claim 8, characterized in that a physiological solution having a concentration of 2 to 5% (w/v) of active principle is prepared.

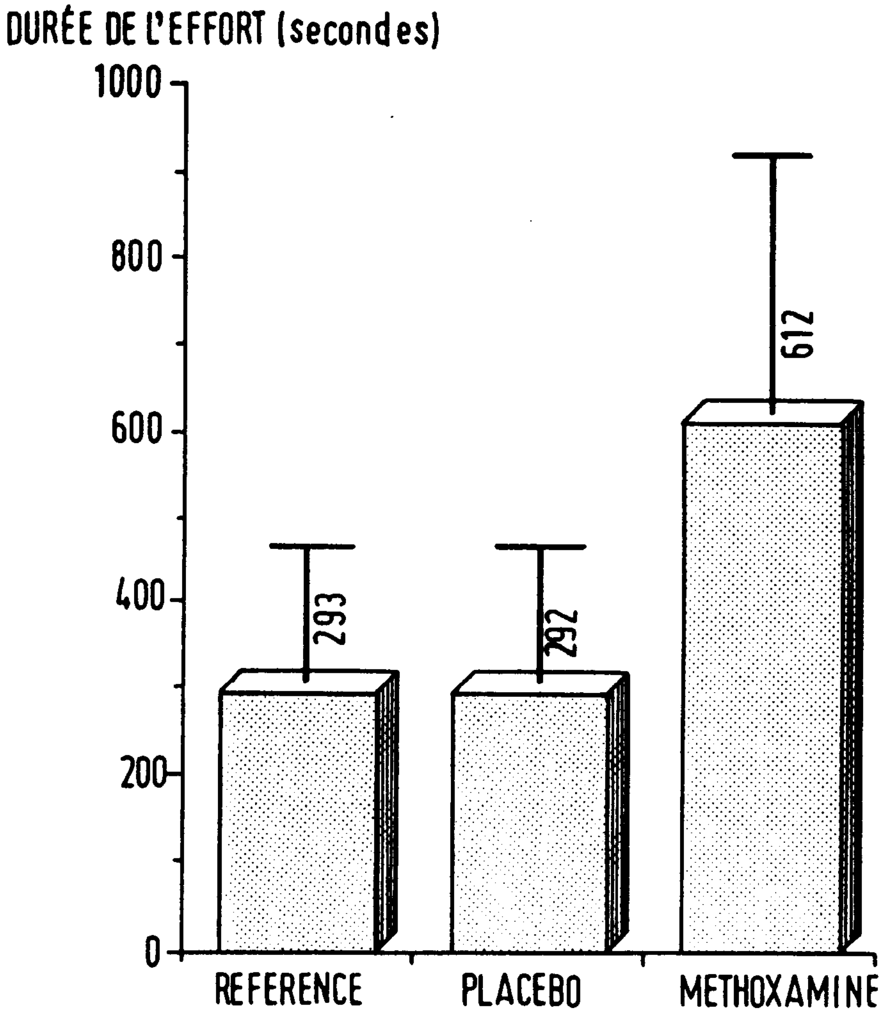
DURÉE DE L'EFFORT (secondes)
1000
800
600
400
200
0
293
292
612
REFERENCE
PLACEBO
METHOXAMINE